# EUROPEAN PATENT APPLICATION

(11) **EP 4 379 056 A1**
(43) Date of publication of application: **05.06.2024**
(21) Application number: 22849838.2
(22) Date of filing: 25.07.2022
(51) Int. Cl.: C12N 15/77, C07K 14/34, C12P 13/14

(54) **MICROORGANISM HAVING WEAKENED ACTIVITY OF LACI FAMILY DNA-BINDING TRANSCRIPTIONAL REGULATOR, AND L-GLUTAMIC ACID PRODUCTION METHOD USING SAME**

(30) Priority: 26.07.2021 KR 20210098072
(71) Applicant: CJ Cheiljedang Corporation, Seoul 04560 (KR)
(72) Inventor: KWON, Nara, Seoul 04560 (KR); LEE, Ah Reum, Seoul 04560 (KR); SONG, Gyuhyeon, Seoul 04560 (KR); LEE, Jin Nam, Seoul 04560 (KR); BONG, Hyun-Ju, Seoul 04560 (KR)
(74) Representative: Bates, Philip Ian
(86) International application number: PCT/KR2022/010905
(87) International publication number: WO 2023/008862

(57) **Abstract**

The application relates to a microorganism having weakened activity of a LacI family DNA-binding transcriptional regulator protein, and an L-glutamic acid production method using same. A Corynebacterium sp. microorganism having weakened activity of a LacI family DNA-binding transcriptional regulator protein has remarkably increased L-glutamic acid productivity, and thus the microorganism can produce L-glutamic acid in a yield higher than that of a conventional microorganism by using same.

## Description

### [TECHNICAL FIELD]

### Cross-reference to related application(s)

The present application claims the benefit of the priority based on Korean Patent application No. 10-2021-0098072 filed on July 26, 2021, and the entire contents disclosed in the document of the corresponding Korean patent application are incorporated as a part of the present description.

The present application relates to a microorganism with weakened activity of a LacI family DNA-binding transcriptional regulator and a production method of L-glutamic acid using the same.

### [BACKGROUND ART]

In order to produce L-amino acids and other useful substances, various studies are being conducted for development of high-efficiency production microorganisms and fermentation process technology. For example, a target substance-specific approach method such as increasing expression of a gene encoding an enzyme involved in L-glutamic acid biosynthesis or removing a gene unnecessary for biosynthesis is mainly used.

However, according to increased demand for L-glutamic acid, there is still a need for research to increase productivity of effective L-glutamic acid.

### [PRIOR ART]

### [PATENT DOCUMENT]

U.S. Patent Publication No. 2011-0027840

### [DISCLOSURE]

### [TECHNICAL PROBLEM]

One object of the present application is to provide a Corynebacterium sp. microorganism with weakened activity of a LacI family DNA-binding transcriptional regulator and enhanced productivity of L-glutamic acid.

Another object of the present application is to provide a production method of L-glutamic acid, comprising culturing the Corynebacterium sp. microorganism in a medium.

### [TECHNICAL SOLUTION]

This will be described in detail as follows. On the other hand, each description and embodiment disclosed in the present application may be applied to each other description and embodiment. In other words, all combinations of various elements disclosed in the present application fall within the scope of the present application. In addition, it cannot be seen that the scope of the present application is limited by the detailed description described below. Furthermore, a number of papers and patent documents are referenced throughout the present description and their citations are indicated. The disclosures of the cited papers and patent documents are incorporate herein by reference in their entirety to more clearly describe the level of the technical field to which the present invention pertains and the content of the present invention.

One aspect of the present application provides a Corynebacterium sp. microorganism (or strain, recombinant cell) with weakened activity of an LacI family DNA-binding transcriptional regulator.

In the present application, the LacI family DNA-binding transcriptional regulator protein may be a protein having LacI family transcriptional regulator activity (for example, LacI family transcriptional regulator), and for example, may be derived from Corynebacterium glutamicum ATCC13869 strain, Corynebacterium glutamicum ATCC13032 strain or Corynebacterium glutamicum ATCC14067 strain, but not limited thereto. In one embodiment, the LacI family DNA-binding transcriptional regulator protein may be derived from Corynebacterium ATCC13869 strain (the sequence may be obtained from GenBank of a known database, NCBI, and for example, it may be GenBank Accession No. WP_060564415.1). In one embodiment, the LacI family DNA-binding transcriptional regulator protein derived from Corynebacterium glutamicum may have or comprise the amino acid sequence of SEQ ID NO: 3, or consist of the amino acid sequence, or be essentially consisting of the amino acid sequence. Specifically, the protein may consist of a polypeptide described by the amino acid sequence of SEQ ID NO: 3.

In one embodiment, the LacI family DNA-binding transcriptional regulator protein may be a polypeptide having LacI family DNA-binding transcriptional regulator activity, which is encoded by an LacI family DNA-binding transcriptional regulator gene, but not limited thereto. In one embodiment, the LacI family DNA-binding transcriptional regulator gene may be derived from Corynebacterium glutamicum ATCC13869 strain, and specifically, it may comprise the nucleic acid sequence of SEQ ID NO: 4 (sequence from the 1,421,016th to 1,422,125th in the nucleic acid sequence of GenBank Accession No. Sequence ID: CP016335.1, for example, BBD29_06680 gene).

In one embodiment, the LacI family DNA-binding transcriptional regulator protein may comprise an amino acid sequence having homology or identity of at least 70%, 75%, 80%, 85%, 90%, 95%, 96%, 97%, 98%, 99%, 99.5%, 99.7% or 99.9% or more to the amino acid sequence described by SEQ ID NO: 3 or consist of the sequence. In addition, as long as it is an amino acid sequence which has this homology or identity and shows efficacy corresponding to the LacI family DNA-binding transcriptional regulator protein, a variant having an amino acid sequence in which some sequences are deleted, modified, substituted, conservatively substituted or added may be comprised in the LacI family DNA-binding transcriptional regulator protein. For example, it is a case which has sequence addition or deletion, naturally occurring mutation, silent mutation or conservative substitution, which does not change the function of the variant of the present application at the N-terminus, C-terminus and/or inside of the amino acid sequence.

The conservative substitution means substituting one amino acid to another amino acid having similar structural and/or chemical properties. This amino acid substitution may generally occur based on similarity in polarity, charge, solubility, hydrophobicity, hydrophilicity and/or amphipathic nature of a residue. Commonly, conservative substitution may hardly affect or not affect activity of a protein or polypeptide.

In the present application, that a polynucleotide or polypeptide "has, comprises a specific nucleic acid sequence (nucleotide sequence) or amino acid sequence, or consists of the sequence, or is essentially consisting of the sequence" may mean that the polynucleotide or polypeptide essentially comprises the specific nucleic acid sequence (nucleotide sequence) or amino acid sequence, and may be interpreted as comprising "a substantially equivalent sequence" in which mutation (deletion, substitution, modification and/or addition) is applied into the specific nucleic acid sequence (nucleotide sequence) or amino acid sequence within a range of maintaining the original function and/or desired function of the polynucleotide or polypeptide. In one embodiment, that a polynucleotide or polypeptide "has or comprises a specific nucleic acid sequence (nucleotide sequence) or amino acid sequence, consists of the sequence, or is essentially consisting of the sequence" may mean that the polynucleotide or polypeptide (i) essentially comprises the specific nucleic acid sequence (nucleotide sequence) or amino acid sequence, or (ii) consists of a nucleic acid sequence or amino acid sequence having homology or identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more to the specific sequence or amino acid sequence, or essentially comprises this, and maintains the original function and/or desired function. In one embodiment, the desired function may mean function of increasing (enhancing) or giving L-glutamic acid productivity of a microorganism.

In the present application, `homology' or 'identity' means a degree of relatedness between two given amino acid sequences or nucleotide sequences, and may be expressed as a percentage. The terms, homology and identity may be often used interchangeably.

The sequence homology or identity of the conserved polynucleotide or polypeptide is determined by a standard arrangement algorithm, and a default gap penalty established by a used program may be used together. Substantially, a homologous or identical sequence may be generally hybridized with all or part of a sequence under a moderately or highly stringent conditions. It is obvious that hybridization also includes hybridization with a polynucleotide containing a general codon or a codon considering codon degeneracy in the polynucleotide.

Whether any two polynucleotide or polypeptide sequences have homology, similarity or identity, may be determined by using a known computer algorithm such as for example, "FASTA" program using a default parameter as Pearson et al (1988) [Proc. Natl. Acad. Sci. USA 85]: 2444. Otherwise, it may be determined by using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM JApplied Math 48: 1073). For example, the homology, similarity or identity may be determined by using BLAST or ClustalW of National biotechnology information database center.

The homology, similarity or identity of the polynucleotide or polypeptide may be determined by comparing sequence information using for example, GAP computer program such as Needleman et al. (1970), J Mol Biol. 48:443, known in for example, Smith and Waterman, Adv. Appl. Math (1981) 2:482. In summary, the GAP program may be defined as a value of dividing the number of similarly arranged symbols (i.e., nucleotides or amino acids) by the total number of symbols in the shorter of two sequences. The default parameter for the GAP program may include (1) binary system comparison matrix (containing values of 1 for identity and 0 for non-identity) and an aggravated comparison matrix of Gribskov et al(1986) Nucl. Acids Res. 14: 6745, disclosed by Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) penalty of 3.0 for each gap and additional 010 penalty for each symbol in each gap (or gap opening penalty 10, gap extension penalty 0.5); and (3) no penalty for a terminal gap.

In the present application, the term, "microorganism (or, strain)" includes all of wild-type microorganisms or naturally or artificially genetically modified microorganisms, and may be a microorganism comprising genetic modification for production of a desired polypeptide, protein or product, as a microorganism with a weakened or enhanced specific mechanism due to a cause such as insertion of a foreign gene or weakening activity of an intrinsic gene.

In the present application, the term, "weakening" of a polypeptide is a concept including all of reducing activity or no activity. The weakening may be used with a term such as inactivation, deficiency, down-regulation, decrease, reduce, attenuation, and the like.

The weakening may also comprise a case in that the activity of the polypeptide itself is reduced or removed compared to the activity of the polypeptide which the original microorganism has due to mutation of a polynucleotide encoding the polypeptide (or protein, for example, LacI family DNA-binding transcriptional regulator protein), and the like, a case in that the overall polypeptide activity level and/or concentration (expression level) is lower compared to a natural strain in a cell due to inhibition of expression of a gene of the polynucleotide encoding this or inhibition of translation into the polypeptide, a case in that expression of the polynucleotide is not done at all, and/or a case in that there is no activity of the polypeptide even if the polynucleotide is expressed. The "endogenous activity" means activity of a specific polypeptide which a parent strain before transformation, or a wild-type or non-modified microorganism originally has, when a trait is changed due to genetic mutation caused by a natural or artificial factor. This may be used with "activity before modification" interchangeably. That the activity of the polypeptide is "inactivated, deficient, reduced, down-regulated, reduced or attenuated" compared to the endogenous activity, means that it is lowered compared to the activity of a specific polypeptide which a parent strain before transformation or non-modified microorganism originally has.

Weakening of activity of this polypeptide (or protein, for example, LacI family DNA-binding transcriptional regulator protein), may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, weakening of a polypeptide (or protein, for example, LacI family DNA-binding transcriptional regulator protein; hereinafter, described as polypeptide), may be
1) deletion of all or part of a gene encoding the polypeptide;
2) modification of an expression regulating region (or expression regulating sequence) so that expression of a gene encoding the polypeptide is reduced;
3) modification of an amino acid sequence composing the polypeptide so that the activity of the polypeptide is removed or attenuated (for example, deletion/substitution/addition of one or more amino acids in the amino acid sequence);
4) modification of the sequence of a gene encoding the polypeptide so that the activity of the polypeptide is removed or attenuated (for example, deletion/substitution/addition of one or more of nucleic acid nucleotides in the nucleic acid nucleotide sequence of the polypeptide gene so that the polypeptide modified to remove or attenuate the activity of the polypeptide is encoded);
5) modification of a nucleotide sequence encoding an initiation codon or 5'-UTR region of gene transcript encoding the polypeptide;
6) introduction of an antisense oligonucleotide (for example, antisense RNA) which complementarily binds to the gene transcript encoding the polypeptide;
7) addition of a complementary sequence to Shine-Dalgarno sequence to the front end of the Shine-Dalgarno sequence of a gene encoding the polypeptide to form a secondary structure in which attachment of ribosome is impossible;
8) addition of a promoter transcribed in an opposite direction at the 3' end of ORF (open reading frame) of a sequence of a gene encoding the polypeptide (Reverse transcription engineering, RTE); or
9) a combination of two or more selected from 1) to 8) above, but not particularly limited thereto.

For example,
the 1) deletion of all or part of the gene encoding the polypeptide may be deletion of the entire polynucleotide encoding the intrinsic target polypeptide in chromosome, substitution into a polynucleotide in which a part of nucleotide is deleted, or substitution into a marker gene.

In addition, the 2) modification of an expression regulating region (or expression regulating sequence), may be mutation occurrence on the expression regulating region (or expression regulating sequence) by deletion, insertion, non-conservative or conservative substitution or a combination thereof, or substitution into a sequence having much weaker activity. The expression regulating region includes a promoter, an operator sequence, a sequence encoding a ribosome binding site and a sequence regulating termination of transcription and translation, but not limited thereto.

Furthermore, the 3) modification of a nucleotide sequence encoding an initiation codon or 5'-UTR region of gene transcript encoding the polypeptide, may be for example, substituting with a nucleotide sequence encoding another initiation codon with a lower polypeptide expression rate compared to the intrinsic initiation codon, but not limited thereto.

In addition, the modification of an amino acid sequence or polynucleotide sequence of the 4) and 5) may be mutation occurrence on the sequence by deletion, insertion, non-conservative or conservative substitution or a combination thereof of an amino acid sequence of the polypeptide or a polynucleotide sequence encoding the polypeptide so as to weaken the activity of the polypeptide, or substitution with an amino acid sequence or polynucleotide sequence modified to have much weaker activity, or an amino acid sequence or polynucleotide sequence modified to have no activity, but not limited thereto. For example, by introducing mutation in a polynucleotide sequence to form a stop codon, expression of a gene may be inhibited or attenuated, but not limited thereto. The "stop codon" is a codon which acts as a signal indicating that does not designate an amino acid and the protein synthesis process is over among codons on mRNA, and in general, 3 kinds of UAA, UAG and UGA may be used as the stop codon.

The microorganism according to one example may be replaced (substituted) with one in which mutation is introduced in a sequence in ORF (open reading frame) of the intrinsic gene of SEQ ID NO: 4 to form a stop codon, and for example, the codon corresponding to the 310th amino acid (for example, glutamine, Gln, Q) of the LacI family DNA-binding transcriptional regulator protein may be replaced (substituted) with a stop codon. For example, the polynucleotide encoding the 310th amino acid, glutamine in the nucleic acid sequence of SEQ ID NO: 4 is "CAG", and it may be one in which mutation of replacing (substituting) "CAG" with "TAA", "TAG" or "TGA", respectively, is introduced to substitute this with a stop codon. In one embodiment, the nucleic acid sequence in which mutation is introduced to form a stop codon may be the nucleic acid sequence of SEQ ID NO: 2 (the 928^{th} C of the wild-type polynucleotide of SEQ ID NO: 4 is substituted with T; C928T).

The 6) introduction of antisense oligonucleotide (for example, antisense RNA) complementarily binding to the gene transcript encoding the polypeptide may refer to for example, the literature [Weintraub, H. et al., Antisense-RNA as a molecular tool for genetic analysis, Reviews - Trends in Genetics, Vol. 1(1) 1986].

The 7) addition of a complementary sequence to Shine-Dalgarno sequence in the front end of Shine-Dalgarno sequence of a gene encoding the polypeptide to form a secondary structure in which attachment of ribosome is impossible may render mRNA translation impossible or reduce the rate.

The 8) addition of a promoter transcribed in an opposite direction at the 3' end of ORF (open reading frame) of a gene sequence encoding the polypeptide (Reverse transcription engineering, RTE) may make an antisense nucleotide complementary to a gene transcript encoding the polypeptide to attenuate the activity.

In the present application, the term, "enhancement" of the polypeptide activity means that the activity of the polypeptide increases compared to the endogenous activity. The enhancement may be interchangeably used with terms such as activation, up-regulation, overexpression, increase and the like. Herein, the activation, intensifying, up-regulation, overexpression and increase may include all exhibiting activity that it does not originally have, or exhibiting improved activity compared to the endogenous activity or activity before modification. The "endogenous activity" means activity of a specific polypeptide, which a parent strain before transformation or a non-modified microorganism originally has, when a trait is changed by genetic mutation by a natural or artificial factor. This may be interchangeably used with "activity before modification". That the activity of the polypeptide is "enhanced", "up-regulated", "overexpressed" or "increased" compared to the endogenous activity means that it is improved compared to the activity and/or concentration (expression level) of a specific polypeptide which a parent strain before transformation or a non-modified microorganism originally has.

The enhancement may be achieved by introducing a foreign polypeptide, or enhancing the activity and/or concentration (expression level) of the intrinsic polypeptide. The enhancement of the activity of the polypeptide may be confirmed from an increase of the level or expression level of the activity of the corresponding polypeptide, or the amount of a product excreted from the corresponding polypeptide.

Various methods well known in the art may be applied for enhancing the activity of the polypeptide, and it is not limited, as long as it can intensify activity of a target polypeptide than a microorganism before modification. Specifically, it may use genetic engineering and/or protein engineering well known to those skilled in the art, which is an ordinary method of molecular biology, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, enhancing the polypeptide of the present application may be
1) increase of the copy number in a cell of a polynucleotide encoding the polypeptide;
2) substitution of a gene expression regulating region on chromosome encoding the polypeptide with a sequence with strong activity;
3) modification of a nucleotide sequence encoding an initiation codon or 5'-UTR region of gene transcript encoding the polypeptide;
4) modification of an amino acid sequence of the polypeptide so that the polypeptide activity is enhanced;
5) modification of a polynucleotide sequence encoding the polypeptide so that the polypeptide activity is enhanced (for example, modification of a polynucleotide sequence of the polypeptide gene so as to encode a polypeptide modified to intensify the activity of the polypeptide);
6) introduction of a foreign polypeptide showing the activity of the polypeptide or a foreign polynucleotide encoding this;
7) codon optimization of a polynucleotide encoding the polypeptide;
8) deformation or chemical modification by selecting an exposed site by analyzing a tertiary structure of the polypeptide; or
9) a combination of 2 or more selected from the 1) to 8), but not particularly limited thereto.

More specifically,
the 1) increase of the copy number in a cell of a polynucleotide encoding the polypeptide may be achieved by introduction of a vector which can be replicated and function regardless of a host, in which a polynucleotide encoding the corresponding polypeptide is operably linked, into a host cell. Otherwise, the polynucleotide encoding the corresponding polypeptide may be achieved by introduction of 1 copy or 2 copies or more in chromosome in the host cell. The introduction in the chromosome may be performed by introducing a vector capable of inserting the polynucleotide into chromosome is introduced into the host cell, but not limited thereto.

The 2) substitution of a gene expression regulating region (or expression regulating sequence) on chromosome encoding the polypeptide with a sequence with strong activity, may be for example, mutation occurrence on the sequence by deletion, insertion, non-conservative or conservative substitution or a combination thereof, or substitution with a sequence having much stronger activity, so as to further intensify the activity of the expression regulating region. The expression regulating region, is not particularly limited thereto, but may comprise a promoter, an operator sequence, a sequence encoding a ribosome binding site, and a sequence regulating termination of transcription and translation, and the like. As one embodiment, it may be substituting the original promoter with a strong promoter, but not limited thereto.

The example of the known strong promoter includes CJ1 to CJ7 promoter (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter and the like, but not limited thereto.

The 3) modification of a nucleotide sequence encoding an initiation codon or 5'-UTR region of gene transcript encoding the polypeptide, may be for example, substituting with a nucleotide sequence encoding another initiation codon with a much higher expression rate of the polypeptide compared to the intrinsic initiation codon, but not limited thereto.

The modification of an amino acid sequence or polynucleotide sequence of the 4) and 5), may be mutation occurrence on the sequence by deletion, insertion, non-conservative or conservative substitution or a combination thereof of an amino acid sequence of the polypeptide or a polynucleotide sequence encoding the polypeptide so as to intensify the activity of the polypeptide, or substitution with an amino acid sequence or polynucleotide sequence modified to have much stronger activity, or an amino acid sequence or polynucleotide sequence modified to increase the activity, but not limited thereto. The substitution may be performed specifically by inserting a polynucleotide into chromosome by homologous recombination, but not limited thereto. Then, the vector to be used may further comprise a selection marker for confirming chromosome insertion.

The 6) introduction of a foreign polypeptide showing the activity of the polypeptide may be introduction of a foreign polynucleotide encoding a polypeptide showing the same/similar activity to the polypeptide into a host cell. The foreign polynucleotide is not limited in its origin or sequence as long as it exhibits the same/similar activity as the polypeptide. The method used for the introduction may be performed by appropriately selecting a known transformation method by those skilled in the art, and the polypeptide is produced by that the introduced polynucleotide is expressed, and thereby, activity thereof may be increased.

The 7) codon optimization of a polynucleotide encoding the polypeptide, may be codon optimization for an endogenous polynucleotide to increase transcription or translation in a host cell, or optimizing a codon thereof for a foreign polynucleotide to achieve optimized transcription and translation in a host cell.

The 8) deformation or chemical modification by selecting an exposed site by analyzing a tertiary structure of the polypeptide, may be for example, deformation or modification by determining a template protein candidate depending on the degree of similarity of the sequence by comparing sequence information of a polypeptide to be analyzed to database in which sequence information of base proteins are stored, and confirming the structure based on this, and thereby, selecting an exposed site to be deformed or chemically modified.

Such enhancing the polypeptide activity, may be that the expression level of the activity or concentration of the corresponding polypeptide is increased on the basis of the activity or concentration of a polypeptide expressed in a wild-type or microorganism strain before modification, or that the amount of the product produced from the corresponding polypeptide is increased, but not limited thereto.

Modification (for example, modification to encode the aforementioned protein variant) of a part or all of a polynucleotide in the microorganism of the present application may be induced by (a) homologous recombination using a vector for chromosome insertion in a microorganism or genome correction using a gene scissor (engineered nuclease, e.g., CRISPR-Cas9) and/or (b) treatment of light such as ultraviolet ray and radioactive ray and/or a chemical substance, but not limited thereto. The method for modifying a part or all of the gene may include a method by DNA recombination technology. For example, by injecting a nucleotide sequence or vector comprising a nucleotide sequence having homology to a target gene into the microorganism to cause homologous recombination, deletion of a part or all of the gene may be achieved. The nucleotide sequence or vector to be injected may comprise a dominant selection marker, but not limited thereto.

In one embodiment, the attenuation of the polypeptide (or protein, for example, LacI family DNA-binding transcriptional regulator protein; hereinafter, described as polypeptide) may be caused by a recombination method. The recombination method may include homologous recombination. When a vector comprising a part of the sequence of a gene encoding the polypeptide is transformed into the microorganism and cultured under presence of a selection marker product, the homologous recombination method may cause homologous recombination of a part of the sequence of the gene with an intrinsic gene in the microorganism.

The microorganism of the present application may be a microorganism in which the LacI family DNA-binding transcriptional regulator protein or a polynucleotide encoding thereof is inactivated or attenuated; or a microorganism (for example, recombinant microorganism) genetically modified by a vector so that the LacI family DNA-binding transcriptional regulator protein or a polynucleotide is inactivated or attenuated, but not limited thereto.

The microorganism (or strain, recombinant cell) of the present application may be a microorganism with L-glutamic acid productivity or enhanced L-glutamic acid productivity (or yield).

The microorganism of the present application may be a microorganism naturally having L-glutamic acid productivity, or a microorganism in which the activity of the LacI family DNA-binding transcriptional regulator protein is weakened and/or the L-glutamic acid productivity is given or improved into a parent strain having no L-glutamic acid productivity, but not limited thereto.

That the microorganism (or strain, recombinant cell) has improved L-glutamic acid productivity (or yield) or has L-glutamic acid productivity may mean that in the microorganism (or strain, recombinant cell), the L-glutamic acid productivity is improved than a non-modified microorganism, a cell before recombination, a parent strain and/or a wild-type strain, or the L-glutamic acid productivity is given different from a non-modified microorganism, a cell before recombination, a parent strain and/or a wild-type strain which has no L-glutamic acid productivity.

The microorganism with attenuated activity of the LacI family DNA-binding transcriptional regulator protein according to one embodiment may have improved (increased) productivity of L-glutamic acid, compared to a homologous non-modified microorganism. In the present application, the "non-modified" microorganism does not exclude a strain comprising mutation which can occur naturally in a microorganism, but it may be a wild-type strain or natural strain itself, or mean a strain before a trait is changed due to genetic mutation caused by a natural or artificial factor. For example, the non-modified microorganism may mean a strain in which the activity of the LacI family DNA-binding transcriptional regulator protein is not attenuated or before it is attenuated according to one embodiment (or strain in which mutation of inducing attenuation of the activity of the LacI family DNA-binding transcriptional regulator protein is not introduced or before it is introduced). The "non-modified microorganism" may be interchangeably used with "strain before modification", "microorganism before modification", "non-mutated strain", "non-modified strain", "non-mutated microorganism" or "standard microorganism". That the activity of the LacI family DNA-binding transcriptional regulator protein is attenuated is as aforementioned. In one embodiment, a target strain for comparing whether the L-glutamic acid productivity is increased, a non-modified microorganism may be Corynebacterium glutamicum ATCC13032 strain, Corynebacterium glutamicum ATCC13869 strain, Corynebacterium glutamicum ATCC14067 strain, a strain in which odhA gene is deleted in the Corynebacterium glutamicum wild type (for example, ATCC13869△odhA strain), or Corynebacterium glutamicum BL2 strain known as L-glutamic acid-producing NTG mutant strain (KFCC11074, Korean Patent No. 10-0292299), but not limited thereto.

The microorganism (or strain, recombinant cell) may further comprise mutation to increase production of L-glutamic acid, and the position of the mutation and/or the type of the gene and/or protein to be mutated may be included without limitation as long as it increases production of L-glutamic acid. The recombinant cell may be used without limitation as long as it is a transformable cell.

As one embodiment, in the microorganism (or strain, recombinant cell) with improved (increased) productivity (or yield), compared to a parent strain before mutated or non-modified microorganism, the L-glutamic acid productivity may be increased by about 1% or more, about 2.5% or more, about 5% or more, about 6% or more, about 7% or more, about 8% or more, about 9% or more, about 10% or more, about 10.5% or more, about 11% or more, about 11.5%or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.4% or more, about 17.5% or more, about 18% or more, about 18.5% or more, about 19% or more, about 19.5% or more, about 20% or more, about 20.5% or more, about 21% or more, about 21.1% or more, about 21.5% or more, about 21.5% or more, about 22% or more, about 22.5% or more, about 23% or more, about 23.5% or more, about 24% or more, about 24.5% or more, about 25% or more, about 25.5% or more, about 26% or more, about 26.5% or more, about 27% or more, about 27.5% or more, about 28% or more, about 28.5% or more, about 29% or more, about 29.5% or more, about 30% or more, about 31% or more, about 32% or more, about 33% or more, about 34% or more, or about 35% or more (the upper limit is not particularly limited, and for example, it may be about 200% or less, about 150% or less, about 100% or less, about 50% or less, about 45% or less, about 40% or less, or about 35% or less), and in one embodiment, it may be increased by about 17.2% or more, about 21.4% or more, or about 21.6% or more. In another embodiment, in the microorganism (or strain, recombinant cell) with increased productivity (or yield), compared to a parent strain before mutated or non-modified microorganism, the L-glutamic acid productivity (or yield) may be increased by about 1.1 time or more, about 1.12 times or more, about 1.13 times or more, 1.15 times or more, 1.16 times or more, 1.17 times or more, 1.18 times or more, 1.19 times or more, about 1.2 times or more, about 1.21 times or more, about 1.22 times or more, 1.25 times or more, or about 1.3 times or more (the upper limit is not particularly limited, and for example, it may be about 10 times or less, about 5 times or less, about 3 times or less, or about 2 times or less), and in one embodiment, it may be increased by about 1.172 times or more, about 1.214 times or more, about 1.216 times or more. More specifically, in the recombinant strain with increased productivity (or yield), compared to a parent strain before mutated or non-modified microorganism, the L-glutamic acid productivity may be increased by about 17.2%, about 21.4%, or about 21.6% (or about 1.17 times, about 1.21 times, or about 1.22 times), but not limited thereto. The term "about" is a range including all of ±0.5, ±0.4, ±0.3, ±0.2, ±0.1, and the like, and includes all values within a range equal to or similar to the value following the term about, but not limited thereto.

In one embodiment, the *Corynebacterium* sp. Microorganism may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* and/or *Corynebacterium flavescens.*

As other one embodiment, the recombinant microorganism of the present application may be a microorganism in which the activity of a part of a protein in a biosynthesis pathway of L-glutamic acid is further enhanced, or the activity of a part of a protein in a degradation pathway of L-glutamic acid is additionally inactivated to intensify the L-glutamic acid productivity.

Specifically, the microorganism of the present application may be a microorganism in which the OdhA protein is further inactivated, or the odhA gene is further deleted. More specifically, the microorganism of the present application may be Corynebacterium glutamicum in which the OdhA protein is inactivated in Corynebacterium glutamicum ATCC13869 or a microorganism in which the odhA gene is deleted in the Corynebacterium glutamicum ATCC13869. The OdhA protein may comprise the amino acid sequence of NCBI Sequence ID WP_060564343.1 (for example, amino acid sequence of SEQ ID NO: 23). The OdhA protein may be a protein having activity of a multifunctional oxoglutarate decarboxylase/oxoglutarate dehydrogenase thiamine pyrophosphate-binding subunit/dihydrolipoyllysine-residue succinyltransferase subunit derived from a Corynebacterium glutamicum strain. The *odhA* gene may be derived from the Corynebacterium glutamicum ATCC13869 strain, and specifically, it may comprise the nucleic acid sequence of SEQ ID NO: 24 (sequence from 1,276,170th to 1,279,787th in the nucleic acid sequence of GenBank Accession No. Sequence ID: CP016335.1, for example, BBD29_06050 gene).

However, the OdhA protein inactivation or *odhA* gene deletion is one kind of examples, and it is not limited thereto, and the microorganism of the present application may be a microorganism in which the protein activity of various known L-glutamic acid biosynthesis pathways is enhanced or the protein activity of degradation pathways is inactivated or weakened.

Another aspect of the present application provides a production method of L-amino acid, comprising culturing the microorganism of the present application in a medium.

The production method of L-amino acid of the present application may comprise culturing the microorganism of the present application in a medium. The microorganism of the present application is as aforementioned.

In addition, the L-amino acid of the present application may be L-glutamic acid.

In the present application, "culturing" means growing the Corynebacterium glutamicum strain of the present application in an appropriately adjusted environmental condition. The culturing process of the present application may be conducted according to an appropriate medium and a culturing condition known in the art. Such a culturing process may be used by easily adjusting it by those skilled in the art depending on a strain to be selected. Specifically, the culturing may be batch, continuous and/or fed-batch, but not limited thereto.

In the present application, "medium" means a substance in which nutrients required for culturing the Corynebacterium glutamicum strain of the present application are mixed as a main component, and supplies nutrients and growth factors and the like, including water essential for survival and growth. Specifically, the medium and other culturing condition used for culturing of the Corynebacterium glutamicum strain of the present application may be used without particular limitation as long as it is a medium used for culturing of a common microorganism, but the Corynebacterium glutamicum strain of the present application may be cultured while adjusting temperature, pH, and the like under an aerobic condition in a common medium containing a carbon source, a nitrogen source, a phosphorus source, an inorganic compound, an amino acid and/or a vitamin, and the like which are suitable.

Specifically, the culturing medium for the Corynebacterium sp. Strain may be found in the literature ["Manual of Methods for General Bacteriology" by the American Society for Bacteriology (Washington D.C., USA, 1981)].

In the present application, the carbon source may include carbohydrate such as glucose, saccharose, lactose, fructose, sucrose, maltose, and the like; sugar alcohol such as mannitol, sorbitol and the like, organic acid such as pyruvic acid, lactic acid, citric acid, and the like; and amino acid such as glutamic acid, methionine, lysine and the like. In addition, a natural organic nutrient such as starch hydrolysate, molasses, blackstrap molasses, rice bran, cassava, sugar cane grounds and corn steep liquid may be used, and specifically, carbohydrate such as glucose and sterilized pre-treated molasses (that is, molasses converted into reduced sugar), and the like may be used, and other appropriate amounts of carbon sources may be variously used without limitation. These carbon sources may be sued alone or in combination of two or more kinds, but not limited thereto.

As the nitrogen source, inorganic nitrogen sources such as ammonia, ammonium sulfate, ammonium chloride, ammonium acetate, ammonium phosphate, ammonium carbonate, ammonium nitrate, and the like; and organic nitrogen sources such as amino acid including glutamic acid, methionine, glutamine, and the like, peptone, NZ-amine, meat extract, yeast extract, malt extract, corn steep liquid, casein hydrate, fish or decomposition product thereof, defatted soybean cake or decomposition product thereof, and the like may be used. These nitrogen sources may be used alone or in combination of two or more kinds, but not limited thereto.

The phosphorus source may comprise potassium monohydrogen phosphate, potassium dihydrogen phosphate, or a sodium-containing salt corresponding thereto, or the like. As the inorganic compound, sodium chloride, calcium chloride, iron chloride, magnesium sulfate, iron sulfate, manganese sulfate, calcium carbonate, and the like may be used, and other amino acids, vitamins and/or appropriate precursors, and the like may be included. These constituents or precursors may be added to a medium in a batch or continuous manner. However, it is not limited thereto.

In addition, during the culturing of the Corynebacterium sp. strain of the present application, a compound such as ammonium hydroxide, potassium hydroxide, ammonia, phosphoric acid, sulfuric acid, and the like may be added to a medium in an appropriate manner to adjust the pH of the medium. Furthermore, during the culturing, an antifoaming agent such as fatty acid polyglycol ester may be used to inhibit bubble generation. In addition, in order to maintain the aerobic condition of the medium, oxygen or oxygen-containing gas was injected into the medium, or in order to maintain the anaerobic and non-aerobic condition, without injection of gas, nitrogen, hydrogen or carbon dioxide gas may be injected, but not limited thereto.

In the culturing of the present application, the culturing temperature may be maintained at 20 to 45°C, specifically, 25 to 40°C, and the culturing may be performed for about 10 to 160 hours, but not limited thereto.

The L-amino acid (for example, L-glutamic acid) produced by the culturing of the present application may be secreted into a medium or remain in a cell.

The production method of L-amino acid of the present application, may further comprise preparing the microorganism (strain) of the present application, preparing a medium for culturing the microorganism, or a combination thereof (in any order), for example, before the culturing.

The production method of L-amino acid of the present application, may further comprise recovering L-amino acid from the medium (medium in which the culturing is performed) or microorganism (Corynebacterium sp. strain) according to the culturing. The recovering may be further comprised after the culturing.

The recovering may collect target L-amino acid by using an appropriate method known in the art according to the culturing method of the microorganism of the present application, for example, a batch, continuous or fed-batch culturing method, and the like. For example, centrifugation, filtration, treatment with a crystallized protein precipitating agent (salting out method), extraction, ultrasonic disruption, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and target L-amino acid may be recovered from the medium or microorganism by using an appropriate method known in the art.

In addition, the production method of L-amino acid of the present application may additionally comprise purifying. The purifying may be performed, by using a suitable method known in the art. In one embodiment, when the production method of L-amino acid of the present application comprises both the recovering and purifying, the recovering and purifying may be performed continuously or non-continuously in any order, or may be performed simultaneously or integrated into one step, but not limited thereto.

Other one aspect of the present application provides a composition for producing an L-amino acid (for example, L-glutamic acid) comprising the microorganism of the present application; a medium culturing thereof; or a combination of 2 or more of them.

The composition of the present application may further comprise any appropriate excipient commonly used in a composition for producing an amino acid, and this excipient may be for example, a preservative, a wetting agent, a dispersing agent, a suspending agent, a buffer, a stabilizer or a tonicifying agent, or the like, but not limited thereto.

In the composition of the present application, the microorganism (strain), medium and L-amino acid, and the like are as described in other aspects above.

Other one aspect of the present application provides a use of the microorganism of the present application; a medium culturing thereof; or a combination of 2 or more of them for producing an L-amino acid (for example, L-glutamic acid).

Other one aspect of the present application provides a use of the microorganism of the present application; a medium culturing thereof; or a combination of 2 or more of them for the manufacture of a composition for producing an L-amino acid (for example, L-glutamic acid).

### [ADVANTAGEOUS EFFECTS]

The Corynebacterium sp. microorganism with weakened activity of the LacI family DNA-binding transcriptional regulator of the present application has significantly increased productivity of L-glutamic acid, and therefore, using this, it is possible to produce L-glutamic acid with a higher yield compared to conventional microorganisms.

### [MODE FOR INVENTION]

Hereinafter, the present application will be described in more detail by examples. However, the following examples are preferable embodiments to illustrate the present application only, and therefore, are not intended to limit the scope of the present application thereto. On the other hand, technical matters not described in the present description can be sufficiently understood and easily implemented by those skilled in the art or similar technical fields of the present application.

### Example 1: Production of vector for expressing LacI family DNA-binding transcriptional regulator protein variant in microorganism

In the present example, in order to confirm the effect on L-glutamic acid production of a variant (Q310*; SEQ ID NO: 1, 309 sequences) in which an codon corresponding to glutamine (Gln, Q) at the 310^{th} position of the LacI family DNA-binding transcriptional regulator protein amino acid sequence (amino acid sequence of SEQ ID NO: 3) was substituted to a stop codon (*), a vector for producing a strain expressing thereof was produced as follows.

Using gDNA (genomic DNA) of wild-type Corynebacterium glutamicum ATCC 13 869 as a template, PCR was performed separately by using the primer pair of the sequences described as SEQ ID NOs: 5 and 6 and the primer pair of the sequences described as SEQ ID NOs: 7 and 8, respectively. Using the mixture of the two fragments obtained above as a template, overlapping PCR was performed again using the primer pair of the sequences of SEQ ID NO: 5 and SEQ ID NO: 8 to obtain a fragment. As polymerase, Solg^{™} Pfu-X DNA polymerase was used, and PCR was performed by denaturation at 95°C for 5 minutes, and then repeating denaturation at 95°C for 30 seconds, annealing at 55°C for 30 seconds and polymerization at 72°C for 1 minute and 30 seconds 30 times, and then polymerization at 72°C for 5 minutes.

The amplified gene fragment and vector pDCM2 for chromosome transformation cut with SmaI restriction enzyme (Korean Patent Publication No. 10-2020-0136813) were cloned using Gibson assembly (DG Gibson et al., NATURE METHODS, VOL.6 NO.5, MAY 2009, NEBuilder HiFi DNA Assembly Master Mix) method, and then, cloning was performed by mixing Gibson assembly reagent and each gene fragment with a calculated number of moles and then preserving them at 50°C for 1 hour. The strain was smeared in an LB solid medium comprising kanamycin (25 mg/l). After selecting a colony in which a targeted gene was inserted, a vector was obtained by using a commonly known plasmid (vector) extraction method. The vector was named pDCM2-BBD29_06680 (Q310*). The primer sequences used in the present example were described in Table 1 below.

**[Table 1]**

| Name | Sequence (5'->3') | SEQ ID NO |
|---|---|---|
| 1F | ATTCGAGCTCGGTACCCGAAAACCCAGAGCTGC TTG | SEQ ID NO: 5 |
| 2R | CATTGATCAGCTTCTaCAGAATCTCAAACGC | SEQ ID NO: 6 |
| 3F | GCGTTTGAGATTCTGtAGAAGCTGATCAATG | SEQ ID NO: 7 |
| 4R | CGACTCTAGAGGATCCCCCCTGTGCCTGCCTGC G | SEQ ID NO: 8 |

### Example 2: Production of L-glutamic acid producing strain derived from wild-type Corynebacterium glutamicum and introduction of LacI family DNA-binding transcriptional regulator protein variant

### Example 2-1: Production of Corynebacterium glutamicum strain having L-glutamic acid productivity derived from wild-type Corynebacterium glutamicum

In order to produce a strain having L-glutamic acid productivity derived from Corynebacterium glutamicum ATCC13869, based on the prior art (Appl Environ Microbiol. 2007 Feb;73(4): 1308-19. Epub 2006 Dec 8.), Corynebacterium glutamicum ATCC13869△*odhA* strain in which *odhA* gene (GenBank Accession No. WP_060564343.1, SEQ ID NO: 24) was deleted was produced.

Specifically, for odhA deletion, using Corynebacterium glutamicum ATCC13869 chromosome DNA as a template, the upstream region and downstream region of the odhA gene were obtained by performing PCR by using the primer pair of SEQ ID NOs: 17 and 18 and the primer pair of SEQ ID NOs: 19 and 20, respectively. As polymerase, SolgTM Pfu-X DNA polymerase was used, and the PCR amplification condition was denaturation at 95 °C for 5 minutes, and then repeating denaturation at 95°C for 30 seconds, annealing at 58°C for 30 seconds, and polymerization at 72°C for 60 seconds 30 times, and then polymerization at 72°C for 5 minutes.

A recombinant vector was obtained by cloning the amplified *odhA* upstream and downstream regions, and vector pDCM2 for chromosome transformation cut with SmaI restriction enzyme using Gibson assembly method, and named as pDCM2-△*odhA*. Cloning was performed by mixing Gibson assembly reagent and each gene fragment with a calculated number of moles and then preserving at 50°C for 1 hour.

The produced pDCM2-△*odhA* vector was transformed into Corynebacterium glutamicum ATCC13869 strain by electroporation, followed by secondary crossover to thereby obtain a strain with a deletion of odhA gene on the chromosome. Deletion of the *odhA* gene was confirmed through PCR using SEQ ID NOs: 21 and 22 and genome sequencing, and the produced strain was named ATCC13869△*odhA*. The sequences of the primers used in the present example were described in Table 2 below.

**[Table 2]**

| Name | Sequence (5'->3') | SEQ ID NO |
|---|---|---|
| 13F | TGAATTCGAGCTCGGTACCCTTGAACGGAATTG GGTGG | SEQ ID NO: 17 |
| 14R | CCCAGGTGGCATCGGTACCTTCACCCAGCGCCA CGCAG | SEQ ID NO: 18 |
| 15R | CGCTGGGTGAAGGTACCGATGCCACCTGGGTTG GTCAAG | SEQ ID NO: 19 |
| 16R | GTCGACTCTAGAGGATCCCCGGACAAGGAATGG AGAGA | SEQ ID NO: 20 |
| 17F | CTTACCGTTGTTGCCCTT | SEQ ID NO: 21 |
| 18R | CTCCTTCACCCACATCATT | SEQ ID NO: 22 |

### Example 2-2: Production of LacI family DNA-binding transcriptional regulator protein variant-introduced strain

The vector pDCM2-BBD29_06680 (Q310*) produced in Example 1 above was transformed into the ATCC13869△*odhA* produced in Example 2-1 above by electroporation, followed by secondary crossover to thereby obtain a strain in which BBD29_06680 (Q310*) mutation was introduced on chromosome. The BBD29_06680 (Q310*) mutation-introduced strain was confirmed by PCR using SEQ ID NOs: 9 and 10 and genome sequencing, and the produced strain was named CA02-1626. The CA02-1626 strain was named Corynebacterium glutamicum CA02-1626, and deposited to Korean Culture of Microorganisms (KCCM) on January 18th, 2021 as accession number KCCM12930P.

The sequences of the primers used in the present example are described in Table 3 below.

**[Table 3]**

| Name | Sequence (5'->3') | SEQ ID NO |
|---|---|---|
| 5F | GCTGCTCGTGAAGCTGG | SEQ ID NO: 9 |
| 6R | GAACCCACCTGAGCATTC | SEQ ID NO: 10 |

### Example 2-3: Comparison of L-glutamic acid productivity of strain expressing LacI family DNA-binding transcriptional regulator protein variant

Using the strain produced in the 2-2, ATCC13869△*odhA* strain as a control group, the L-glutamic acid productivity was to be confirmed. The control group and CA02-1626 strain were cultured by the method as below.

Each strain was inoculated in a 250mf corner-baffled flask containing a seed medium 25mℓ, and was cultured with shaking at 200rpm, at 30°C for 20 hours. After that, seed culture solution of 1mℓ was inoculated in a 250mi corner-baffled flask containing a production medium 25mℓ, and was cultured with shaking at 200rpm, at 30°C for 40 hours. After completing the culturing, the yield of L-glutamic acid was measured using high-performance liquid chromatography (HPLC), and the measured result was shown in Table 4 below.

### <Seed medium>

Glucose 1%, beef extract 0.5%, polypeptone 1%, sodium chloride 0.25%, yeast extract 0.5%, urea 0.2%, pH 7.2

### <Production medium>

Raw sugar 6%, calcium carbonate 5%, ammonium sulfate 2.25%, potassium monophosphate 0.1%, magnesium sulfate 0.04%, iron sulfate 10 mg/L, thiamine hydrochloride 0.2 mg/L, biotin 50/*µ*g/L

**[Table 4]**

| Strain name | L-GLUTAMIC ACID concentration (g/L) | L-GLUTAMIC ACID concentration increase rate (%) |
|---|---|---|
| ATCC13869Δ*odhA* | 1.90 | - |
| CA02-1626 | 2.31 | 21.6 |

As shown in Table 4 above, it was confirmed that the concentration of L-glutamic acid increased by about 21.6% in the CA02-1626 strain, in which BBD29_06680(Q310*) mutation was introduced, compared to the control group, ATCC13869△*odhA* strain.

### Example 3: Production of LacI family DNA-binding transcriptional regulator protein-deleted strain and measurement of L-glutamic acid productivity

### Example 3-1: Production of LacI family DNA-binding transcriptional regulator protein gene-deleted vector

In the example, it was confirmed that the productivity of L-glutamic acid was improved, when a codon corresponding to glutamine (Gln, Q) which is the 310^{th} amino acid of the LacI family DNA-binding transcriptional regulator protein was substituted with a stop codon. Accordingly, in the present example, the effect on production of L-glutamic acid of the LacI family DNA-binding transcriptional regulator (BBD29_06680) gene deletion was to be confirmed.

Specifically, for BBD29_06680 deletion, using gDNA (genomic DNA) of Corynebacterium glutamicum ATCC13869 as a template, the upstream region and downstream region of the BBD29_06680 gene were obtained by performing PCR using the primer pair of SEQ ID NOs: 11 and 12 and the primer pair of the sequences described as SEQ ID NOs: 13 and 14. As polymerase, SolgTM Pfu-X DNA polymerase was used, and the PCR amplification condition was denaturation at 95 °C for 5 minutes, and then repeating denaturation at 95°C for 30 seconds, annealing at 58°C for 30 seconds and polymerization at 72°C for 60 seconds 30 times, and then polymerization at 72°C for 5 minutes. A recombinant vector was obtained by cloning the amplified DNA fragment using vector pDCM2 for transforming chromosome cut with SmaI restriction enzyme and Gibson assembly method, and was named pDCM2-△BBD29_06680. The cloning was performed by mixing Gibson assembly reagent and each gene fragment with a calculated number of moles and then preserving them at 50°C for 1 hour.

The sequences of the primers used in the present example are described in Table 5 below.

**[Table 5]**

| Name | Sequence (5'->3') | SEQ ID NO |
|---|---|---|
| 7F | attcgagctcggtacccCCAGTTCGGTCACAAGAC | SEQ ID NO: 11 |
| 8R | GCTTTTTGGGCTGCTTCGCTTCTTCGGGCTGG | SEQ ID NO: 12 |
| 9F | CCAGCCCGAAGAAGCGAAGCAGCCCAAAAAGC | SEQ ID NO: 13 |
| 10R | GACTCTAGAGGATCCCCGGACAACGCCTTGGCG | SEQ ID NO: 14 |

### Example 3-2: Production of LacI family DNA-binding transcriptional regulator protein-deleted strain

The vector pDCM2-△BBD29_06680 produced in Example 3-1 above was transformed into the ATCC13869△*odhA* produced in Example 2-1 above by electroporationfollowed by secondary crossover to thereby obtain a strain in which the BBD29_06680 gene was deleted on chromosome, and this was confirmed by PCR using the primer pair of SEQ ID NOs: 15 and 16 and genome sequencing. The selected strain was named CA02-1627. The sequences of the primers used in the present examples were described in Table 6 below.

**[Table 6]**

| Name | Sequence (5'->3') | SEQ ID NO |
|---|---|---|
| 11F | GCAAGGCGATGGAACGTC | SEQ ID NO: 15 |
| 12R | CTCATCCAAGTGGTGCG | SEQ ID NO: 16 |

### Example 3-3: Measurement of L-glutamic acid productivity of LacI family DNA-binding transcriptional regulator protein-deleted strain

In order to confirm the L-glutamic acid productivity using the ATCC13869△*odhA* strain produced in Example 2-1 above as a control group, evaluation was conducted according to the fermentation titer evaluation method of Example 2-3, and after completing the culturing, the yield of L-glutamic acid was measured using high-performance liquid chromatography (HPLC), and the measured result was shown in Table 7 below.

**[Table 7]**

| Strain name | L-GLUTAMIC ACID concentration (g/L) | L-GLUTAMIC ACID concentration increase rate (%) |
|---|---|---|
| ATCC13869Δ*odhA* | 1.92 | - |
| CA02-1627 | 2.33 | 21.4 |

As shown in Table 7 above, it was confirmed that the concentration of L-glutamic acid increased by about 21.4% in the CA02-1627, in which the BBD29_06680 gene was deleted, compared to the control group, ATCC13869△*odhA* strain.

### Example 4: Production of NTG mutant-derived LacI family DNA-binding transcriptional regulator protein variant-introduced strain and measurement of L-glutamic acid productivity

In order to confirm whether the BBD29_06680 (Q310*) variant showed the same effect even in the strain derived from NTG (N-Methyl-N'-nitro-N-nitrosoguanidine) mutation Corynebacterium sp. with increased L-glutamic acid productivity, the variant was introduced into Corynebacterium glutamicum BL2 strain known as an L-glutamic acid producing NTG mutation strain (KFCC11074, Korean Patent No. 10-0292299).

The pDCM2-BBD29_06680 (Q310*) vector produced in Example 1 was transformed into the KFCC11074 strain by electroporation, followed by secondary crossover to thereby select a strain in which the BBD29_06680 (Q310*) variant was introduced on chromosome. This was confirmed by PCR using SEQ ID NOs: 9 and 10 and genome sequencing, and the produced strain was named CA02-1630.

For the produced CA02-1630 and Corynebacterium glutamicum KFCC11074 strain, a fermentation titer experiment was conducted by the method specified below.

Each strain was inoculated in a 250 mℓ corner-baffled flask containing a seed medium 25 mF, and was cultured with shaking at 200 rpm, at 30 °C for 20 hours. After that, seed culture solution of 1 mℓ was inoculated in a 250 mℓ corner-baffled flask containing a production medium 25 mF, and was cultured with shaking at 200 rpm, at 30 °C for 40 hours. After completing the culturing, the yield of L-glutamic acid was measured by the method using HPLC, and the measured result was shown in Table 8 below.

### <Seed medium>

Glucose 1%, beef extract 0.5%, polypeptone 1%, sodium chloride 0.25%, yeast extract 0.5%, urea 0.2%, pH 7.2

### <Production medium>

Raw sugar 6%, calcium carbonate 5%, ammonium sulfate 2.25%, potassium monophosphate 0.1%, magnesium sulfate 0.04%, iron sulfate 10 mg/L, thiamine hydrochloride 0.2 mg/L, biotin 500*µ*g/L

**[Table 8]**

| Strain name | L-GLUTAMIC ACID concentration (g/L) | L-GLUTAMIC ACID concentration increase rate (%) |
|---|---|---|
| KFCC11074 | 6.91 | - |
| CA02-1630 | 8.10 | 17.2 |

As shown in Table 8 above, it was confirmed that the concentration of L-glutamic acid increased by about 17.2% in the CA02-1630 strain compared to the control group, KFCC11074 strain.

From the above description, those skilled in the art to which the present application pertains will be able to understand that the present application may be conducted in other specific forms without changing the technical spirit or essential characteristics thereof. In this regard, it should be understood that the examples described above are illustrative and not restrictive in all respects. The scope of the present application should be construed as including all changed or modified forms derived from the meaning and scope of the claims to be described later and equivalent concepts rather than the detailed description above.

### [ACCESSION NUMBER]

Name of depository authority : Korean Culture Center of Microorganisms
Accession number : KCCM12930P
Accession date : 20210118

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA02-1626 | Accession number given by the
INTERNATIONAL DEPOSITARY AUTHORITY:
KCCM12930P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 18. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 18. 2021

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A *Corynebacterium* sp. microorganism with weakened activity of LacI family DNA-binding transcriptional regulator protein.

2. The microorganism according to claim 1, wherein the LacI family DNA-binding transcriptional regulator protein is derived from the genus of *Corynebacterium.*

3. The microorganism according to claim 1, wherein the LacI family DNA-binding transcriptional regulator protein consists of a polypeptide described as the amino acid sequence of SEQ ID NO: 3.

4. The microorganism according to claim 1, wherein the LacI family DNA-binding transcriptional regulator protein is encoded by a polypeptide described as the nucleotide sequence of SEQ ID NO: 4.

5. The microorganism according to claim 1, wherein the *Corynebacterium* sp. microorganism is *Corynebacterium glutamicum.*

6. The microorganism according to claim 1, wherein the *Corynebacterium* sp. microorganism has increased productivity of L-glutamic acid compared to a parent strain or wild type in which the activity of the LacI family DNA-binding transcriptional regulator protein is not weakened.

7. A production method of L-glutamic acid, comprising culturing a *Corynebacterium* sp. microorganism with weakened activity of LacI family DNA-binding transcriptional regulator protein in a medium.

8. The production method of L-glutamic acid according to claim 7, further comprising recovering L-glutamic acid from the medium or microorganism according to the culturing.

9. The production method of L-glutamic acid according to claim 7, wherein the LacI family DNA-binding transcriptional regulator protein is derived from the genus of *Corynebacterium.*

10. The production method of L-glutamic acid according to claim 7, wherein the LacI family DNA-binding transcriptional regulator protein consists of a polypeptide described as the amino acid sequence of SEQ ID NO: 3.

11. The production method of L-glutamic acid according to claim 7, wherein the LacI family DNA-binding transcriptional regulator protein is encoded by a polypeptide described as the nucleotide sequence of SEQ ID NO: 4.

12. The production method of L-glutamic acid according to claim 7, wherein the *Corynebacterium* sp. microorganism is *Corynebacterium glutamicum.*

13. The production method of L-glutamic acid according to claim 7, wherein the *Corynebacterium* sp. microorganism has increased productivity of L-glutamic acid compared to a parent strain or wild type in which the activity of the LacI family DNA-binding transcriptional regulator protein is not weakened.
